# EUROPEAN PATENT SPECIFICATION

(11) **EP 2 687 212 B1**
(45) Date of publication and mention of the grant of the patent: **08.08.2018**
(21) Application number: 12772023.3
(22) Date of filing: 10.04.2012
(51) Int. Cl.: A61K 31/353, A61K 35/63, A61P 9/10, A61P 25/00, A61P 43/00

(54) **COMBINATIONS OF DIHYDROQUERCETIN AND DRONE BROOD FOR USE IN THE TREATMENT OF ISCHAEMIC STROKE**
KOMBINIERUNGEN AUS DIHYDROQUERCETIN UND DROHNENBRUT ZUR BEHANDLUNG ISCHÄMISCHER SCHLAGANFÄLLE
COMBINAISONS DE LA DIHYDROQUERCÉTINE ET DU COURVAIN DE FAUX-BOURDONS DESTINÉES AU TRAITEMENT DES ACCIDENTS ISCHÉMIQUES CÉRÉBRAUX

(43) Date of publication of application: 22.01.2014
(73) Proprietor: Obshestvo S Ogranichennoj Otvetstvennostju "PARAFARM", Penza 440026 (RU)
(72) Inventor: TRIFONOV, Vyacheslav Nikolaevich, Penzenskaja obl. 442960 (RU); ELISTRATOVA, Julia Anatoljevna, Penza 440026 (RU); ELISTRATOV, Konstantin Gennadievich, Penza 440061 (RU); KURUS, Natalia Vyacheslavovna, Penza 440061 (RU)
(74) Representative: Engel, Christoph Klaus
(86) International application number: PCT/RU2012/000270
(87) International publication number: WO 2012/141615

(56) References cited:
- EP-A2- 2 730 286
- RU-C1- 2 233 666
- RU-C2- 2 357 746
- RU-C2- 2 410 979

## Description

The invention relates to the field of medicine, and more specifically, to the pharmaceutical industry involved in manufacturing medical products, and can be used in restoration of brain function following ischemic stroke, as well as in prevention of strokes, therapeutic and preventive treatment of cerebral vessels, improving the quality of thinking, memory, adaptation to physical and mental loads, and improving cerebral metabolism.

There are known therapeutic agents for post ischemic stroke recovery and prevention of recurrent strokes, such as actovegin or mexidol that improve cerebral microcirculation. The drawback of these known medications is their narrowly focused action aimed solely at improvement of cerebral microcirculation. An equally important objective is to strengthen blood vessels and capillary walls to prevent recurrent strokes and ensure the recovery of brain cells by providing them with an adequate supply of nutrients, and to eliminate toxins and waste products of cell activity from the intercellular substance (Alekseyev, A.A. -Integrative (Systemic/Family) Connective-Tissue Medicine. V. 1-4, M.: URSS, 2005).

It is an object of this invention to expand the range and provide increased pharmacological activity of post-stroke medications.

This object is achieved by the provision of a composition for use in the treatment or prevention of ischemic stroke, wherein the composition comprises:
Dihydroquercetin - 10 mg to 400 mg,
Drone brood - 40 mg to 1000 mg.

These quantity limits are based on the following:
For drone brood:
The maximum intake of drone brood is s 1000 mg as determined by its applicability.

The minimum intake of drone brood is 40 mg (Patent of Russian Federation No. 2233666) as determined by its pharmacological activity.

For dihydroquercetin:
The maximum intake of dihydroquercetin is 400 mg as determined by its applicability.

The minimum intake of dihydroquercetin is 10 mg as determined by its pharmacological activity.

The use of the said mixture of dihydroquercetin and drone brood has not been described in the literature. The novelty of the claimed invention lies in the fact that a mixture of said substances:
1) is claimed for the first time to be used for restoration of brain function following ischemic stroke, as well as in prevention of thereof.
2) shows higher pharmacological activity than that of individual ingredients of the mixture.

These properties are not obvious to a skilled person from the prior art.

Dihydroquercetin is a reference antioxidant. It has strong anti-inflammatory and anti-allergenic properties; provides the strengthening and repair of connective tissue; lowers cholesterol levels; potentiates effects of many essential substances (such as vitamin C and vitamin E); strengthens blood vessels and capillaries; improves microcirculation; prevents blood clotting; reduces inflammatory changes within the cells, boosts the immune system and promotes cell regeneration. It increases the body tissues' resistance to damage caused by excessive blood glucose levels and lowers the risk of developing diabetes and alleviates the progression of existing diabetes, has favorable effects on the nervous system and activates neural processes.

Drone brood is characterized by a large number of functional groups of enzymes, sulphide groups, as well as testosteroid hormones, progesterone and estradiol. Due to such complex of constituents, drone brood promotes fast recovery of brain cells and increases metabolic rate. The chemical composition of drone brood includes: proteins (10-20%); carbohydrates (1-5.5%); fats (5-6.3%); amino acids (11.4%); glucose (3.18-5%); fructose, sucrose (up to 0.5%); micronutrients (mg%): K - 0.50, Na - 38, Ca - 14, P - 189, Mg - 2, Fe - 3.23, Mn - 4.40, Zn - 5.54, Cu - 2, Cr, Co, Ni, Ag, Au, etc.; vitamins (water and fat soluble): A - 0.54 IU/g: xanthophyll - 0.297 mg %; B-carotene - 0.426 IU/g; B2 - 0.739 mg %; D - 950 IU/g; choline - 442.8 mg %; nicotinic acid - 15.8 mg %, ascorbic acid, vitamin E.

The synergy of action is explained as follows: Vitamins E and A contained in drone brood potentiate the action of dihydroquercetin; the use of dihydroquercetin without them is unjustified because of the considerable decrease in capillary permeability. Improved capillary permeability makes it possible to provide cells with necessary nutrients contained in drone brood, and boost the intercellular metabolism. Capillaries do not directly supply brain cells with necessary nutrients. Nutrients from capillary blood get to the loose connective tissue called intercellular substance. Nerve endings and lymph capillaries also exit into this open peri-capilllary space, and waste products of cells are removed from this space through veins. Brain cells destroyed by an ischemic stroke intoxicate the intercellular space, the released toxins damaging the weakened cells and causing their death. Therefore, the recovery of the intercellular space plays an essential role in the recovery of brain cells. This process is assisted by drone brood containing large amounts of nicotinic acid and 15 fatty acids (myristic, pentadecanoic, palmitic, palmitoleic, margaric, stearinic, oleic, linoleic, gamma-linolenic, arachic, eicosenoic, docosanoic, docosenoic, tetracosanoic and nervonic). These acids promote metabolic processes and accelerate the decomposition of lipids and their elimination from the body. These acids act as precursors in the synthesis of prostaglandins, the latter performing regulatory functions in the intercellular metabolism.

Therefore, the synergistic effect consists in:
1) potentiating the action of dihydroquercetin, namely the properties of increasing capillary permeability and strengthening the vessel walls, by the constituents of drone brood, in the absence of which the effect of dihydroquercetin is much weaker. It should be noted that capillaries themselves also receive nutrients from intercellular space and, therefore, the use of drone brood enhances the vitality of capillaries, which cannot be achieved by the use of dihydroquercetin alone.
2) enhancing of metabolic processes within the intercellular space of the brain brought about by the constituents of drone brood. Without the use of dihydroquercetin the intercellular metabolism worsens considerably due to reduced microcirculation (inflow /outflow).

Example procedures for making the product.

### Example 1

Dihydroquercetin powder is taken in the amount of 10 kg and mixed with 80 kg of lyophilized drone brood. The resulting mixture is blended until homogeneous and the resulting product is encapsulated.

### Example 2

Dihydroquercetin powder is taken in the amount of 100 kg and mixed with 400 kg of lyophilized drone brood. The resulting mixture is then blended until homogeneous and pressed into tablets.

The following formulation was chosen for experiments: dihydroquercetin - 100 mg, lyophilized drone brood - 100 mg.

Experiments were performed on male outbred white rats with the body weight of 350 to 450 g. The animals were trained to acquire a conditioned passive avoidance reflex (CRPA) in an apparatus consisting of a well-lit suspended platform connected to a dark chamber provided with an electrified floor. In the training process, a rat guided by the inborn hole-exploratory behavior entered the dark chamber and received an electric footshock. The passive avoidance memory was assessed 24 hours after the training by registering the number of animals not entering the dark chamber for a period of 180 seconds, and measuring the latency time for those rats that entered it. The study showed that rats usually avoid entering the dangerous chamber or enter it towards the end of the test, with an average latency time of 158±12.1 sec.

Subsequently, brain ischemia was induced by ligating the left carotid artery in rats anesthetized with chloral hydrate (400 mg/kg, i.p.) followed by medial cerebral artery occlusion (MCAO) performed under a microscope proximal of its bifurcation into frontal and parietal branches.

The animals were then randomized into 4 groups:
1) Control group,
2) Animals of the 2^{nd} group were treated with dihydroquercetin administered intraperitoneally 30 minutes, and subsequently, 2, 24 and 48 hours after the surgery.
3) Animals of the 3^{rd} group were treated with lyophilized drone brood powder administered intraperitoneally 30 minutes, and subsequently, 2, 24 and 48 hours after the surgery.
4) Animals of the 4^{th} group were treated with the claimed mixture administered intraperitoneally 30 minutes, and subsequently, 2, 24 and 48 hours after the surgery.

Animals of the control group were treated with normal saline (0.9% sodium chloride solution) at the same time points.

The described surgery results in massive ischemic damage to the cerebral cortex localized within its frontoparietal and dorso-lateral regions. The total volume of ischemic damage was 18.65±2.65% in saline-treated controls.
1 Control rats treated with normal saline showed latency times of 20±6.1 sec (most animals did not remember the footshock experienced the day before and quickly entered the dangerous chamber).
2 The group of ischemized rats treated with dihydroquercetin showed an increase in latency times to 25±3.3, which suggests improvement in memory function.
3 The group of ischemized rats treated with lyophilized drone brood powder showed an increase in latency times to 35±6.3, which suggests improvement in memory function.
4 The group of ischemized rats treated with the claimed mixture showed an increase in latency times to 62±5.1, which suggests improvement in memory function.

The above said proves that separate use of individual ingredients of the claimed product does not show such pharmacodynamics, in contrast to the claimed product.

## Claims

1. A composition for use in the treatment or prevention of ischemic stroke, wherein the composition comprises:
- dihydroquercetin - 10 mg to 400 mg;
- drone brood - 40 mg to 1000 mg.

## Patentansprüche

1. Rezeptur zur Verwendung bei der Behandlung oder Prävention von ischämischen Schlaganfällen, wobei die Rezeptur beinhaltet:
- Dihydroquercetin - 10 mg bis 400 mg;
- Drohnenbrut - 40 mg bis 1000 mg.

## Revendications

1. Composition pour une utilisation dans le traitement ou la prévention de l'accident ischémique cérébral, dans laquelle la composition comprend :
- de la dihydroquercétine - entre 10 mg et 400 mg ;
- du couvain de mâles - entre 40 mg et 1000 mg.
